(19) Europäisches Patentamt
European Patent Office
Office européen des brevets

(11) Numéro de publication : **0 522 936 A1**

# (12) DEMANDE DE BREVET EUROPEEN

(21) Numéro de dépôt : **92401902.9**

(22) Date de dépôt : **03.07.92**

(51) Int. Cl.[5] : **A61K 47/10**, A61K 47/26, A61K 47/44

(30) Priorité : **08.07.91 FR 9108527**

(43) Date de publication de la demande : **13.01.93 Bulletin 93/02**

(84) Etats contractants désignés : **PT**

(71) Demandeur : **RHONE-POULENC RORER SA**
**20, avenue Raymond Aron**
**F-92160 Antony (FR)**

(72) Inventeur : **Bastart, Jean-Pierre**
**12 rue des Bergères**
**F-77150 Lesigny (FR)**
Inventeur : **Dupechez, Thierry**
**2 Avenue Paul Doumer**
**F-91360 Villemoisson Sur Orge (FR)**
Inventeur : **Fabre, Jean-Louis**
**9 rue Fagon**
**F-75013 Paris (FR)**

(74) Mandataire : **Le Pennec, Magali et al**
**RHONE-POULENC RORER SA, Direction des Brevets, 20 Avenue Raymond Aron**
**F-92165 Antony Cédex (FR)**

(54) **Nouvelles compositions à base de dérivés de la classe des taxanes.**

(57) La présente invention concerne de nouvelles compositions à base de dérivés de la classe des taxanes constituées de solutions de ces dérivés dans un agent tensioactif.
Ces solutions sont utilisées pour préparer des solutés de perfusions.

EP 0 522 936 A1

La présente invention concerne une nouvelle forme pharmaceutique à base d'un agent thérapeutique ayant une activité antitumorale et antileucémique. Elle concerne plus particulièrement une nouvelle forme injectable contenant des produits de la famille des taxanes tels que notamment le taxol ou un de ses analogues ou dérivés de formule générale suivante:

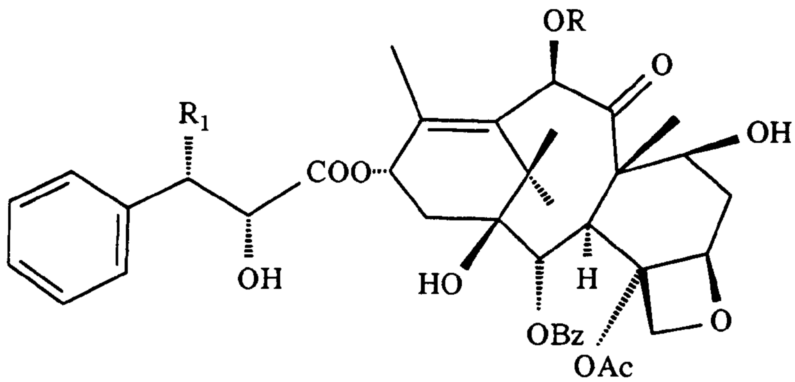

(I)

Dans la formule (I), R représente un atome d'hydrogène ou un radical acétyle, le symbole $R_1$ représente un radical tertiobutoxycarbonylamino ou benzoyloxyamino. On préfère parmi l'ensemble de ces dérivés les deux dérivés pour lesquels R représente un groupe acétyle et $R_1$ un groupe benzoyloxyamino ou celui pour lequel R représente un atome d'hydrogène et $R_1$ un radical tertio butoxycarbonylamino.

Le premier de ces deux composés est plus connu sous la dénomination de taxol, le deuxième est connu sous la dénomination de Taxotère.

Ces produits présentent in vivo une activité importante sur les tumeurs malignes ce qui a permis de les étudier dans le traitement des maladies résistantes à toutes les autres thérapies anticancéreuses.

Malheureusement ces produits présentent une solubilité dans l'eau tellement faible qu'il a été nécessaire de préparer une formulation pour préparation injectable à base d'agent tensioactif et d'éthanol. L'éthanol, est le meilleur solvant qui permette de solubiliser les molécules répondant à la formule (I).

A titre d'exemple, selon la publication de Rowinsky, Lorraine, Cazenave et Donehower parue dans le Journal of the National Cancer Institute, vol. 82, No 15, pages 1247 à 1259, le 1er Août 1990, on prépare une première solution, dite "solution mère", contenant environ 6 mg/ml de taxol dans un mélange solvant composé de:

- 50 % en volume d'éthanol
- 50 % en volume de Crémophor EL.

Lors de l'injection, cette solution est mélangée avec un liquide de perfusion contenant du chlorure de sodium ou du dextrose. Pour obtenir un mélange stable, d'un point de vue physique comme d'un point chimique, les auteurs de cet article disent qu'il faut limiter la concentration en principe actif dans le soluté de perfusion à des concentrations d'environ 0,03 à 0,6 mg/ml (voir publication précédente page 1251 colonne 1, troisième paragraphe).

Or il est souhaitable de pouvoir injecter des doses suffisantes de principe actif, pour cela les cliniciens désirent injecter des concentrations en principe actif comprises entre environ 0,3 et 1 mg/ml dans le liquide de perfusion, au delà de ces doses apparaissent des phénomènes de chocs anaphylactiques difficiles à maîtriser dus pour l'essentiel au Cremophor (voir la publication de Rowinsky page 1250 deuxième colonne dernier paragraphe).

Toujours selon cette publication, pour obtenir de telles concentrations (entre 0,3 et 1mg/ml) il est nécessaire d'injecter des solutions contenant en même temps que le principe actif des concentrations en chacun des composés suivants, éthanol et surtout Crémophor, d'environ 8 g pour 100 ml de soluté. Le traitement demandant souvent l'administration de doses élevées de principe actif et la concentration du principe actif dans la solution étant relativement faible l'injection de fort volume a pour effet de provoquer durant le traitement en plus des manifestations anaphylactiques des manifestations d'éthylisme.

Il a été découvert par la mise en oeuvre des formes pharmaceutiques de la présente invention que l'on pouvait soit diminuer fortement les concentrations en éthanol, soit encore supprimer totalement le Crémophor et l'éthanol dans les perfusions.

Pour cela, selon un premier procédé de mise en oeuvre de l'invention, on prépare une solution mère contenant le principe actif dans un mélange de solvants composé d'éthanol qui est le meilleur solvant biocompatible des principes actifs de la classe des taxanes et d'un agent tensioactif choisi parmi les polysorbates commercialisés notamment sous la dénomination Tween, les esters de polyoxyéthylènes glycols commercialisés par exemple sous la dénomination d'Emulphor, ou les esters de polyéthylèneglycol et d'huiles de ricin commercialisées par exemple sous la dénomination de Crémophor.

La solution mère est préparée par dissolution du principe actif dans l'éthanol puis addition progressive de l'agent tensioactif. On peut ainsi préparer des solutions contenant 10 à 100 mg/ml de principe actif dans un mélange contenant environ 50 % d'agent tensioactif.

L'objet de la présente invention est le suivant: l'éthanol meilleur solvant du principe actif peut être presque entièrement supprimé.

Pour préparer, selon le premier procédé de mise en oeuvre de l'invention, les solutions à faible teneur en éthanol, on dissout le principe actif dans l'éthanol comme décrit précédemment puis on ajoute un agent tensioactif quelconque choisi parmi ceux cités précédemment ou d'autres, permettant après la dilution en milieu aqueux, la formation de micelles contenant le principe actif encapsulé dans l'agent tensioactif. L'éthanol contenu dans cette solution est ensuite éliminé au moins partiellement par évaporation sous vide ou par tout autre moyen approprié.

Selon un deuxième procédé de préparation de la solution mère, on dissout directement le principe actif dans l'agent tensioactif. Selon une meilleure manière de mettre en oeuvre l'invention, on prépare une solution de tensioactif contenant notamment 1 à 2 % d'éthanol et on ajoute en continu le principe actif dans cette solution en agitant à l'aide par exemple d'un broyeur hélicoïdal ou d'une turbine dilacératrice. La présence d'une faible quantité d'éthanol apporte plusieurs avantages, le milieu présente une viscosité moins élevée, le mouillage de la poudre est amélioré ainsi que la filtration finale de la solution.

La solution mère, à faible teneur en éthanol, contient de préférence moins de 5 % d'éthanol, elle contient encore plus préférentiellement moins de 2 % d'éthanol. Cette solution est stable et peut ainsi contenir jusqu'à 200 mg/ml et de préférence jusqu'à 80 mg/ml de principe actif dans l'agent tensioactif.

La solution mère de taxol présente encore plus préférentiellement une concentration comprise entre 6 et 20 mg/ml de principe actif dans l'agent tensioactif. Cette solution est mélangeable, notamment, selon une concentration comprise entre 0,1 et 1 mg par millilitre avec le liquide de perfusion, que ce soit un soluté physiologique ou une solution de glucose. Les perfusions préparées à partir des solutions mères précédentes, à faible teneur en éthanol contiennent encore plus préférentiellement entre 0,3 et 0,5 mg/ml de taxol et contiennent moins de 1 ml/l d'éthanol.

La perfusion de taxol contenant le principe actif sans éthanol présente une stabilité physique comprise entre 8 et une centaine d'heures. On entend par stabilité physique le fait que la solution ne présente aucun critère visible de précipitation après 8 à 10 heures de conservation à température ambiante.

La solution mère de Taxotère présente de préférence une concentration comprise entre 20 et 80 mg/ml de principe actif dans l'agent tensioactif. Cette solution est mélangeable, notamment, selon une concentration comprise entre 0,1 et 0,5 mg par millilitre avec le liquide de perfusion, que ce soit un soluté physiologique ou une solution de glucose. Les perfusions préparées à partir des solutions mères précédentes, à faible teneur en éthanol contiennent encore plus préférentiellement entre 0,1 et 0,3 mg/ml de Taxotère; elles contiennent moins de 15 ml/l d'agent tensioactif et moins de 1 ml/l d'éthanol.

La perfusion de Taxotère contenant le principe actif sans éthanol présente une stabilité physique pouvant atteindre plusieurs mois.

Les perfusions de taxol ou de Taxotère sont ensuite injectées à l'homme à un débit prédéterminé en fonction de la quantité de principe actif que l'on veut injecter. On n'observe pas avec ces solutions les phénomènes de chocs anaphylactiques que l'on observait avec les solutions de l'art antérieur.

Ainsi ces dernieres perfusions ont permis de diminuer, par rapport à l'art antérieur, les quantités d'agent tensioactif injectées à l'homme d'environ 80 % et les quantités d'éthanol de presque 100 %.

L'invention sera plus complètement décrite à l'aide des exemples suivants qui ne doivent pas être considérés comme limitatifs de l'invention.

## EXEMPLE COMPARATIF SELON L'ART ANTERIEUR

On dissout 0,180 g de taxol dans 15 ml d'éthanol. On complète avec du Crémophor pour obtenir 30 ml d'une solution qui contient 6 mg/ml de taxol.

Cette solution est diluée dans un soluté de perfusion de glucose à 5% à raison de 1 mg/ml, le soluté de perfusion contient 87,7 ml/l de Cremophor et 87,7 ml/l d'éthanol.

La solution de perfusion est stable pendant plus de 21 heures.

EXEMPLES SELON L'INVENTION

On dissout 32 g de Taxotère dans 340 ml d'éthanol absolu puis on ajoute 830 g de Polysorbate 80. On évapore l'éthanol au rotavapor à 30°C sous une pression de 15 mm de mercure pendant 2 heures.

La solution obtenue est stable, elle contient 40 mg/ml de Taxotère.

Après dilution dans une solution de perfusion de glucose à 5 %, à des concentrations de 0,1; 0,3 et 0,5 mg/ml on observe la stabilité des solutions obtenues.

On reproduit le même procédé à partir d'une solution contenant 60 mg/ml de Taxotère.

On reproduit le même essai à partir de solutions de taxol contenant 12 et 20 mg/ml de taxol.

Les résultats sont indiqués dans le tableau 1.

TABLEAU 1 SELON L'INVENTION

| produit | Solvant | Solution mère concentration | principe actif dans la perfusion | Tensioactif dans la perfusion | Ethanol dans la perfusion | Stabilité |
|---|---|---|---|---|---|---|
| taxol | Polysorbate | 20 mg/ml | 1 mg/ml | 50 ml/l | <0,3 ml/l | >8H |
| taxol | Polysorbate | 20 mg/ml | 0,3 mg/ml | 15 ml/l | <0,09 ml/l | >24H |
| taxol | Polysorbate | 12 mg/ml | 1 mg/ml | 83,3 ml/l | <0,5 ml/l | >48H |
| Taxotère | Polysorbate | 40 mg/ml | 0,5 mg/ml | 11,6 ml/l | 0,09 ml/l | 8H-23H |
| Taxotère | Polysorbate | 40 mg/ml | 0,3 mg/ml | 6,9 ml/l | 0,05 ml/l | 8H-23H |
| Taxotère | Polysorbate | 40 mg/ml | 0,1 mg/ml | 2,3 ml/l | 0,02 ml/l | 29H-45H |
| Taxotère | Polysorbate | 60 mg/ml | 0,1 mg/ml | 1,5 ml/l | <0,01 ml/l | 8H-23H |

EXEMPLE 8

Dans un réacteur en acier, on introduit 258 g de Taxotère que l'on dissout avec 2425 g d'éthanol sous agitation mécanique pendant 45 minutes.

On ajoute 6156 g de polysorbate 80 et on homogénéise sous agitation mécanique pendant 15 minutes.

On transfère la solution dans un réacteur et on distille l'alcool sous une pression réduite de 10 à 50 millibar (1000 à 5000 Pa) la température étant maintenue entre 18 et 28°C.

On distille l'alcool jusqu'à ce que sa teneur soit inférieure à 2 %.

La solution obtenue est filtrée sur un filtre ayant une dimension de pore de 0,2 μm. Elle contient :

- 1,3 % d'éthanol
- 39,6 mg/ml de Taxotère.

Après dilution à 1 mg/ml dans une poche de perfusion contenant du glucose à 5 % la solution est stable sans précipitation apparente pendant une période supérieure à 2 mois.

EXEMPLE 9

On dissout 160 g de Taxotère ou 160 mg de taxol dans 10 ml d'un mélange d'éthanol absolu Chrémophor EL(218), on évapore l'éthanol au rotovapor à 30°C sous pression de 25 mn de mercure pendant 3 heures.

Les solutions obtenues sont stables. Elles contiennent 20 mg/ml de Taxotère ou de taxol. Après dilution dans une soluté de perfusion de glucose à 5 % à des concentrations de 0,1 et 0,5 mg/ml, on observe une précipitation entre 30 et 95 heures.

EXEMPLE 10

On dissout dans une fiole erlenmeyerde 500 ml 275.5 g de polysorbate 80 et 5.4 g d'éthanol absolu puis on agite avec un barreau magnétique jusqu'à homogénéisation complète du mélange.

Dans une fiole de 50 ml on charge 26.13 g de la solution préparée précédemment. On met la fiole dans un bain marie chauffé auparavant et maintenu pendant toute la durée de l'essai à 30°C. On agite à environ 600 tours par minute avec un barreau aimenté.

Avec une spatule on ajoute 1.076 g de Taxotère en plusieurs fractions de façon à faire disparaitre les grumeaux entre deux ajouts (durée de l'opération: environ une heure). Après incorporation de la dernière fraction de Taxotère, maintenir l'agitation jusqu'à ce que la solution devienne limpide (durée de l'opération: environ deux heures).

## Revendications

**-1-** Nouvelles compositions à base d'un dérivé de formule (I)

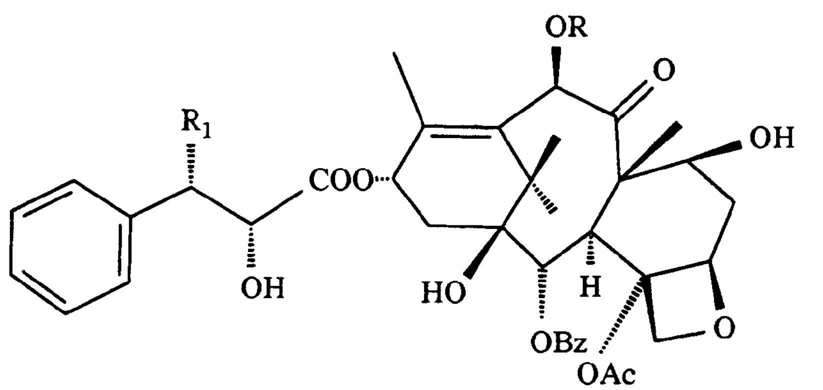

(I)

dans laquelle R représente un atome d'hydrogène ou un radical acétyle, le symbole $R_1$ représente un radical tertiobutoxycarbonylamino ou benzoyloxyamino en solution dans un agent tensioactif choisi parmi les polysorbates, les esters de polyoxyéthylène glycols et les esters de polyéthylène glycol et d'huiles de ricin hydrogénées presque exemptes d'éthanol.

**-2-** Compositions selon la revendication 1 caractérisées en ce qu'elles contiennent moins de 5% d'éthanol, et de préférence moins de 2% d'éthanol

**-3-** Compositions selon l'une quelconque des revendications 1 ou 2 caractérisées en ce que dans le composé de formule (I) R représente l'hydrogène et $R_1$ un radical tertiobutoxycarbonylamino.

**-4-** Compositions selon l'une quelconque des revendications 1 ou 2 caractérisées en ce que dans le composé de formule (I) R représente un groupe acétyle et $R_1$ représente un radical benzoyloxyamino.

**-5-** Compositions selon l'une quelconque des revendications 1 à 4 caractérisées en ce qu'elles contiennent jusqu'à 200 mg/ml et de préférence jusqu'à 80 mg/ml de composés de formule (I).

**-6-** Compositions selon les revendications 3 et 5 caractérisées en ce qu'elles contiennent 20 à 80 mg/ml de composés de formule (I).

**-7-** Compositions selon les revendications 4 et 5 caractérisées en ce qu'elles contiennent 6 à 20 mg/ml de composés de formule (I).

**-8-** Procédé de préparation des compositions selon quelconque des revendications précédentes caractérisé en ce que l'on solubilise le principe actif dans l'éthanol, on ajoute l'agent tensioactif puis on évapore l'éthanol.

**-9-** Procédé de préparation des compositions selon l'une quelconque des revendications 1 à 7 caractérisé en ce que la composition est préparée par addition lente du principe actif à une solution de l'agent tensioactif contenant 1 à 2% d'éthanol.

**-10-** Perfusion caractérisée en ce qu'elle contient moins de 0,5 mg/ml et de préférence 0,1 à 0,3 mg/ml de composé selon la revendication 3 et qu'elle contient moins de 1 ml/l d'éthanol et moins de 15 ml/l d'agent tensioactif.

**-11-** Perfusion caractérisée en ce qu'elle contient 1 mg/ml ou moins de composé selon la revendication 4 et qu'elle contient moins de 1 ml/l d'éthanol.

Office européen des brevets

# RAPPORT DE RECHERCHE EUROPEENNE

Numero de la demande

EP 92 40 1902

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | JOURNAL OF THE NATIONAL CANCER INSTITUTE, vol. 82, no. 15, 1 août 1990, pages 1247-1259; E.K. ROWINSKY et al.: "Taxol: a novel investigational antimicrotubule agent"<br>* Page 1251, colonne de gauche: "Pharmaceutical data" *<br>--- | 1-11 | A 61 K 47/10<br>A 61 K 47/26<br>A 61 K 47/44 |
| A | EP-A-0 253 738 (RHONE-POULENC SANTE)<br>* Revendications 1,4; page 8, lignes 1-16; page 8, exemple *<br>--- | 1-11 | |
| A | STN INTERNATIONAL INFORMATION SERVICES, BASE DE DONNEES: CHEMICAL ABSTRACTS, vol. 106, no. 22, 1987, abrégé no. 182581c, Columbus, Ohio, US; B.D. TARR et al.: "A new parenteral vehicle for the administration of some poorly water soluble anti-cancer drugs", & J. PARENTER. SCI. TECHNOL., 41(1), 31-3<br>* Abrégé *<br>--- | 1-11 | DOMAINES TECHNIQUES RECHERCHES (Int. Cl.5) |
| A | EP-A-0 118 316 (LIPID SPECIALITIES)<br>* Revendications 1,19,21; page 14, exemple 10 *<br>----- | 1-11 | A 61 K |

Le présent rapport a été établi pour toutes les revendications

| Lieu de la recherche | Date d'achèvement de la recherche | Examinateur |
|---|---|---|
| LA HAYE | 21-09-1992 | SCARPONI U. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)